# EUROPEAN PATENT APPLICATION

(11) **EP 4 162 926 A1**
(43) Date of publication of application: **12.04.2023**
(21) Application number: 20940212.2
(22) Date of filing: 08.06.2020
(51) Int. Cl.: A61K 8/96, A61Q 19/02, A61Q 19/08

(54) **METHOD FOR EXTRACTING EXTRACT OF LIQUOR PRODUCTION BY-PRODUCT AND COMPOSITION FOR WHITENING, WRINKLE IMPROVEMENT AND ANTI-AGING COMPRISING THE EXTRACT ACCORDING TO THE METHOD**

(71) Applicant: Kim, Sang Wook, Busan 49424 (KR)
(72) Inventor: Kim, Sang Wook, Busan 49424 (KR)
(74) Representative: Byrne, Declan
(86) International application number: PCT/KR2020/007372
(87) International publication number: WO 2021/251508

(57) **Abstract**

Provided are a method for extracting an extract of liquor production by-products and a composition for whitening, wrinkle improvement and anti-aging effects, which includes the extract obtained by the above method. The extract of liquor production by-products according to the above method has excellent whitening, wrinkle improvement or anti-aging effects and thus can be used as a material for cosmetics and the like.

## Description

### [Technical Field]

The present invention relates to a method for extracting by-product in production of alcoholic beverages and a composition for whitening, wrinkle improvement and anti-aging effects, which includes the extract obtained by the above method. Specifically, the present invention relates to a method for fermenting and extracting by-products generated after alcoholic beverage production, and a composition for whitening, wrinkle improvement and anti-aging effects that includes the extract obtained by the above extraction method.

### [Background Art]

Alcoholic beverage is divided into fermented liquor, distilled liquor, and other liquor (hybrid liquor) according to the manufacturing methods. Fermented liquor is an alcoholic beverage made by fermenting sugar or starch contained in fruits, grains and other raw materials by the action of mold and yeast. This alcoholic beverage has relatively low alcohol content, as well as unique aroma and soft taste resulting from ingredients of the raw material. Wine, beer, makgeolli (Korean raw-rice wine), herbal liquor, Korean refined rice wine, etc. are included therein. Distilled liquor is the liquor obtained by distilling fermented liquor or mash, and has a relatively high alcohol concentration, wherein some or most of the impurities can be removed depending on the distillation method. In particular, soju made by dilution of spirit, which is consumed a lot in Korea, is generally made from spirits having almost removed impurities, specifically, brandy, whiskey, vodka, rum and tequila belong to this type of liquor. Other liquor (hybrid liquor) is an alcoholic beverage made by leaching or distilling brewed or distilled liquor with addition of fruits, spices, sweeteners, and medicinal herbs, specifically, ginseng liquor, plum wine, Siberian ginseng wine, gin, various cocktails, and the like are belong to this type of liquor.

Alcoholic beverage consumption in Korea is very high, and various types of alcoholic beverages are produced and sold. However, there is a problem in that it is not easy to process a large amount of by-products generated after the production of alcoholic beverages. Therefore, there is a need for a plan to utilize the by-products generated during the production of alcoholic beverages as a high value-added industry.

Meanwhile, the popularity of Korean cosmetics is increasing due to the recent Hallyu (K-culture) craze, and the market size is also expanding. However, it is not easy to overcome the barriers of products of foreign countries such as France and United States, etc., which are leading countries in cosmetic technology due to high technological barriers. Therefore, in order to overcome the barriers of products of the above countries, the need for developing functional cosmetics or raw materials thereof is increasing.

In the prior art using by-products generated during alcoholic beverage production, Korean Patent Registration Publication No. 10-0737862 (Functional organic fertilizer containing lees as a by-product of alcoholic beverage production, as well as novel Bacillus sp. strain), Korean Patent Laid-Open Publication No. 10-2010-0103751 (Fermentation of tomato fermented wine and liquid feed manufacturing method using fermentation by-products), Korean Patent Registration Publication No. 10-1402568 (Method for manufacturing distilled spirits based on byproduct of wine), and Korean Patent Registration Publication No. 10-1438587 (Method for manufacturing wine by-product makgeolli and makgeolli manufactured thereby), and Korean Patent Registration Publication No. 10-1763358 (Medium composition and manufacturing method using makgeolli or its by-products) have been known. However, technologies capable of providing by-products generated during liquor production as a raw material for high-functional cosmetics are not yet known, therefore, the need for development of the above technologies is emerging.

### [Prior Art Document]

### [Patent Document]

Korean Patent Registration Publication No. 10-0737862
Korean Patent Laid-open Publication No. 10-2010-0103751
Korean Patent Registration Publication No. 10-1402568
Korean Patent Registration Publication No. 10-1438587
Korean Patent Registration Publication No. 10-1763358

### [Summary of Invention]

### [Problem to be Solved by Invention]

In order to solve the above problems and promote the development of high value-added industry using by-products generated during production of alcoholic beverages, the present invention is intended to provide a method for manufacturing a high-functional raw material using the by-products, and a composition prepared by the manufacturing method.

Accordingly, an object of the present invention is to provide a method for extracting an extract by fermenting by-product in production of alcoholic beverages, and a composition for whitening, wrinkle improvement and anti-aging effects, which includes the extract obtained by the above method.

### [Means for Solving Problems]

In order to achieve the above object, according to an aspect of the present invention, there is provided,
a method for extracting an extract of by-product in production of alcoholic beverages ("liquor production by-product"), comprising:
(1) drying the liquor production by-product;
(2) crushing the by-product dried in the above (1);
(3) fermenting the by-product crushed in the above (2) ; and
(4) extracting the by-product fermented in the above (3) .

Further, there is provided a composition for whitening, wrinkle improvement and anti-aging effects, which includes the extract prepared by the above extraction method.

Further, there is provided a scrub composition obtained by micro-beading the by-product generated in the extraction step (4) of the extraction method.

The by-products generated during production of alcoholic beverages in the present invention are a concept including not only fruits but also seeds, stems, skins, etc. thereof, which are raw materials of the alcoholic beverage, and the form of the by-products may include a solid form or a solid content, but it is not limited thereto.

The alcoholic beverage may include fermented liquor, distilled liquor, and other liquors (hybrid liquors), and is preferably the fermented liquor.

The fermented liquor refers to the alcoholic beverage made by fermenting sugar or starch contained in fruits, grains, and other raw materials by the action of mold and yeast, and may include wine, beer, makgeolli (Korean raw-rice wine), yakju (herbal wine), and cheongju (Korean refined-rice wine), but it is not limited thereto.

Fruits able to become raw materials for the alcoholic beverage may include apples, grapes, strawberries, bokbunja (Raspberry), plums, lemons, and the like, but they are not limited thereto.

The grains may include wheat, rice, brown rice, barley, malt, and the like, but they are not limited thereto.

The drying step (1) may include drying at 15 to 35 °C, preferably at 20 to 30 °C.

When drying at a temperature lower than the above temperature range, oxidation of the raw material may occur due to an increase in drying time. Further, if drying at a temperature higher than the above temperature range, it may cause thermal damage to a phenolic compound, which is a functional component in the raw material.

Further, the drying time may range from 12 to 60 hours, and preferably 24 to 48 hours. If it is less than the above drying time range, a large amount of impurities may be included during extraction. Further, if it exceeds the above drying time range, moisture loss may be increased and the active ingredient may not be extracted.

In the crushing step (2), the by-product may be crushed to a particle size of 0.1 to 3 mm or less, and preferably 0.1 to 2 mm or less.

If the particle size is out of the above range, there is a risk that effective ingredients in the raw material may not be sufficiently eluted or fine particles may not be stably filtered.

In the fermentation step (3), for smooth fermentation, sugar may be supplemented in an amount of 0.1 to 20% by weight of a total weight of the by-products, and the supplemented sugar may include sucrose or glucose, but it is not limited thereto.

If an amount of the supplemented sugar is less than the above range, fermentation may not proceed properly. Further, if it exceeds the above range, there is a risk of over-fermentation.

After sugar supplement, a fermentation strain is added and then distilled water (or purified water) is further added to the by-product, followed by stationary culture or shaking culture at 25 to 50 °C for 24 to 72 hours, resulting in fermentation.

The fermentation strain is not limited, but preferably includes: *Weissella cibaria, Issatchenkia orientalis, Saccharomyces cerevisiae,* and more preferably, *Issatchenkia orientalis* and *Saccharomyces cerevisiae* are introduced.

The fermentation strain may be used for fermentation in an amount of 0.005 to 3% by weight, and preferably 0.01 to 2% by weight based on the total weight including the liquor production by-products and an extraction solvent. When fermenting with the strain in an amount of less than the above range, the fermentation may not be carried out properly. Further, when fermenting with the strain in an amount of more than the above range, it may have a bad (foul) smell.

The extraction solvent used in the extraction step (4) is not limited, but preferably, water or 70% (v/v) ethanol is used for extraction.

Water or ethanol used in the extraction may be added by 3 to 10 times the total volume of the fermented by-product, and preferably is added by 5 times. The extraction may be conducted at room temperature (10 to 50 °C) for 12 to 36 hours, and preferably for 24 hours.

The by-product remaining after extracting the extract in (4) and filtering the remaining fermented broth may be pulverized into particles (micro-beads) in a particle size of 100 to 500 µm, preferably 200 to 400 um, and thereby providing a scrub composition.

The present invention further provides a method for manufacturing an extract of liquor production by-products, which further includes (5) filtering and then concentrating the extract, and (6) pulverizing the extract concentrate,
in addition to the previously described manufacturing method.

In the filtration and concentration step (5), the filtration may be changeable according to conditions such as a filter paper, membrane filter, centrifugation and the like. Further, the concentration may be performed using a vacuum rotary concentrator, and may include concentrating at 45 to 65 °C, and preferably at 50 to 60 °C.

The solids are concentrated to 5 to 60 brix°, and preferably 5 brix° to 30 brix°.

If it is concentrated to a concentration lower than the above range, there is a concern about secondary fermentation by sugar-tolerant strains. Further, if it is concentrated at a concentration higher than the above range, a loss rate during the manufacturing process is increased, and there is a concern that a large amount of impurities are included.

Powdering in step (6) may include both freeze-drying and spray-drying, but it is not limited thereto.

The composition for whitening, wrinkle improvement and anti-aging effects according to the present invention may be a cosmetic composition or a food composition, and preferably the cosmetic composition.

### [Advantageous Effects]

The present invention may provide a method for production of an extract by fermenting and extracting by-products (juice solids) generated during the production of alcoholic beverages, specifically, fermented liquor such as wine, and the alcoholic beverage by-product extract prepared according to the above method may have excellent whitening, wrinkle improvement or anti-aging effects, therefore, can be used as a material for cosmetics and the like.

### [Brief Description of Drawings]

FIG. 1 illustrates a cell culture process.
FIG. 2 illustrates an MTT assay measurement process for measuring cytotoxicity.
FIG. 3 shows results of the cytotoxicity test.
FIG. 4 illustrates a process of measuring the tyrosinase inhibitory activity.
FIG. 5 shows measurement results of the tyrosinase inhibitory activity according to the present invention.
FIG. 6 illustrates a process of measuring the inhibition of melanin production.
FIG. 7 shows measurement results of the inhibition of melanin production according to the present invention.
FIGS. 8A and 8B show evaluation results of reactive oxygen species inhibitory effects on B16F10 melanocytes and CCRF S-180II fibroblasts according to the present invention.
FIGS. 9A and 9B show wrinkle improvement effects based on the expression levels of MMP1 and MMP8 according to the present invention.

### [Mode for Carrying out Invention]

Hereinafter, the present invention will be described in detail by way of examples and experimental examples.

However, the following examples and experimental examples are merely illustrative of the present invention, and the content of the present invention is not limited to the following examples and experimental examples.

### <Examples 1 to 3, and Comparative Examples 1 to 3>

In an embodiment of the present invention, wine juice solids, which are by-products of wine production, were used. The by-products were dried at 15 to 35 °C for 24 to 48 hours, and the dried by-products were crushed to a particle size of 2 mm or less. Thereafter, using sucrose or glucose, sugar was supplemented in an amount of 10% by weight based on the total weight of the by-product.

*Weissella cibaria* strain in Example 1, *Issatchenkia orientalis* strain in Example 2, and *Saccharomyces cerevisiae* strain in Example 3, respectively, were used for treatment of the crushed by-products. Then, fermentation was performed by adding distilled water 10 times the volume of the by-products, followed by stationary culture or shaking culture at 25 to 40 °C for 24 to 72 hours.

The amount of strain used herein was 0.02% by weight based on the total weight including the by-products and water.

Comparative Example 1 is a normal group without any treatment, Comparative Example 2 is a negative control group treated with a stimulant, and Comparative Example 3 is a group treated with normal grape extract. After adding distilled water 5 times the volume of the by-product to the fermented by-products, the extraction process was performed at room temperature for 24 hours in order to yield an extract.

**[TABLE 1]**

| Comparativ e Example 1 | Comparativ e Example 2 | Comparativ e Example 3 | Example 1 | Example 2 | Example 3 |
|---|---|---|---|---|---|
| Untreated group | Negative control group (stimulant treatment) | General grape extract treatment | *Weissella cibaria* treatment | *Issatchenk ia orientalis* treatment | *Saccharomy ces cerevisiae* treatment |

### <Experimental Example 1> Measurement of cytotoxicity of fermented wine extract

### 1-1. Cell culture

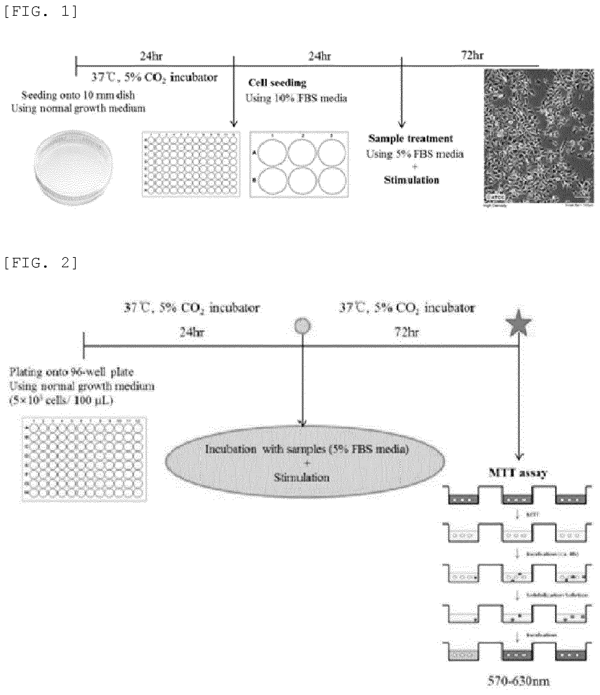

Cells used herein are mouse melanoma B16F10 cells, which were purchased from the Korea Cell Line Bank (KCLB), and the medium used herein is DMEM (Dulbecco's Modified Eagle Medium, gibco, USA) supplemented with 10% FBS (fetal bovine serum, gibco, USA), sodium bicarbonate (Sigma, USA) and 1% Penicilin. Specifically, the cells were cultured using the above medium in an incubator under 37 °C and 5% CO₂ conditions, and the culture process is shown in FIG. 1.

### 1-2. Cytotoxicity measurement

In order to confirm the cytotoxicity of a sample to cells, the strains in Examples 1 to 3 and Comparative Example 3 were used for treatment at concentrations of 400, 500 and 800 ppm, respectively. Thereafter, 700 (v/v) ethanol of 5 times the volume of the fermented wine juice solids was added, followed by extraction for 24 hours at room temperature and measurement by MTT assay. The specific method is as follows.

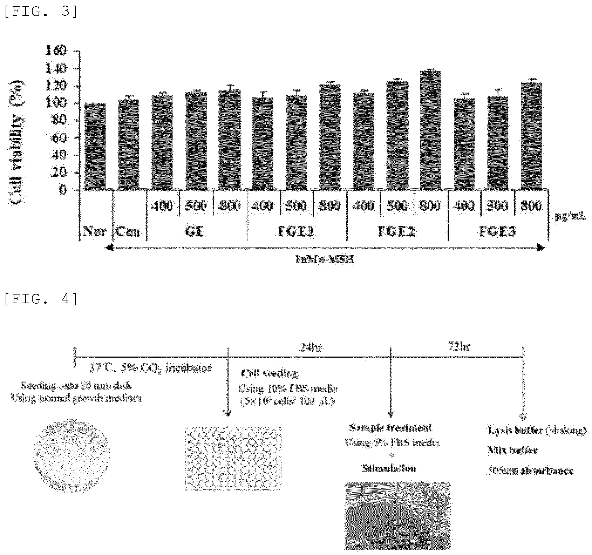

Mouse melanoma B16F10 cells were dispensed by 100 µL in a 96-well plate using DMEM medium containing 10% FBS so as to reach 5×10³ cells/well, and cultured in an incubator under 37 °C and 5% CO₂ conditions. After culturing for 24 hours for cell adhesion, the cells were treated with 150 µL of each diluted sample in the medium supplemented with 5% FBS, followed by exchanging the medium. Then, after 3 hours, 2 nM α-MSH stimulant was supplied. After 72 hours from incubation, each well was treated with 7.5 µL of 5 mg/mL MTT reagent and the medium was removed after 3 hours, followed by adding 100 µL of DMSO to measure absorbance at 570/630 nm (FIG. 2). Cytotoxicity was indicated by the percentage as compared to the control, and comparison results are shown in FIG. 3.

As a result of the experiment, all of the treatments using the strains in Comparative Examples 2 and 3 and Examples 1 to 3, respectively, exhibited similar cytotoxicity to the normal group. Therefore, it is determined that treatment with the strains in Examples 1 to 3 and application of the same to a human body are not harmful.

### <Experimental Example 2> Evaluation of whitening effects through measurement of tyrosinase inhibitory activity

In order to evaluate the whitening effects of the inventive composition, the strains in Comparative Example 3, Examples 1 to 3 and Comparative Examples 1 and 2 were used for treatment at 400 and 800 ppm, respectively, to measure tyrosinase inhibitory activity, and the specific method is as follows.

In order to evaluate the tyrosinase inhibitory activity, 100 µL of mouse melanoma B16F10 cells were dispensed in a 96-well plate using a medium containing 10% FBS to reach 5×10³ cells per well, and cultured in an incubator at 37°C, 5% CO₂ for 24 hours.

Thereafter, the cells were treated with 150 µL of each sample diluted using a medium supplemented with 5% FBS, followed by exchanging the medium. Then, after 3 hours, 2 nM α-MSH stimulant was supplied, and the cells were cultured in an incubator at 37°C, 5% CO₂ for 3 days. After incubation, the medium was removed and the product was treated with 25 µL of lysis buffer containing 1% sodium deoxycholate, Triton X-100, and 100 mM PMSF, followed by shaking for 30 minutes.

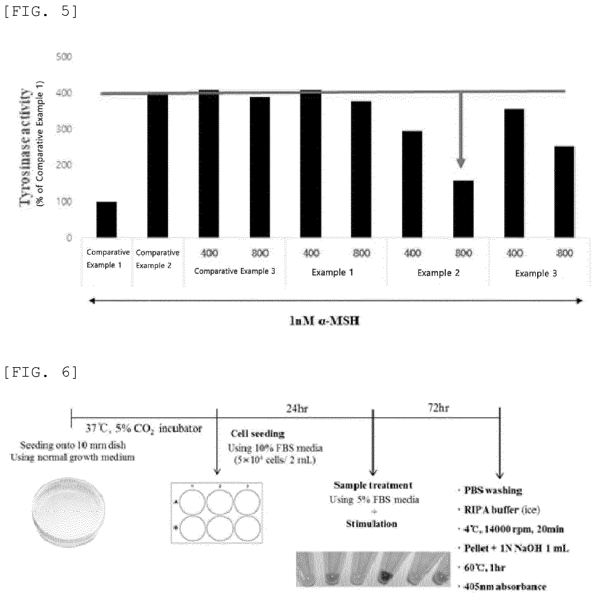

After 30 minutes, Solution A (2% N,N-dimethyl formamide in 100 mM sodium phosphate), Solution B (5 mM L-DOPA in 100 mM sodium phosphate) and Solution C (20 mM MBTH in H2O) were mixed at a ratio of 2:1:1 to produce a mix buffer, and then 140 µL of the mix buffer was added to each well to induce a reaction, followed by measurement of absorbance at 505 nm, and measurement results are shown in Table 2 below and FIG. 5.

**[TABLE 2]**

| | Compar ative Exampl e 1 | Comparativ e Example 2 | | Example 1 | | Example 2 | | Example 3 | |
|---|---|---|---|---|---|---|---|---|---|
| Fermente d strain | - (norma l group) | Negative control group (stimulant treatment) | | *Weissella cibaria* | | *Issatchenk ia orientalis* | | *Saccharomyce s cerevisiae* | |
| Treatmen t concentr ation (ppm) | - | 400 | 800 | 400 | 800 | 400 | 800 | 400 | 800 |
| Tyrosina se activity (%) | 100 | 576 | 638 | 609 | 640 | 281 | 225 | 468 | 399 |

From the result of the experiment, it could be confirmed that, as a result of treatment in Examples 1 to 3 of the present invention after introduction of the stimulant to B16F10 melanoma cells, the tyrosinase activity was inhibited in Examples 1 to 3. In particular, when Example 2 was used for treatment at a concentration of 800 ppm, the tyrosinase activity was inhibited and measured to about 1/3 level as compared to the negative control group in Comparative Example 2 at the same concentration, therefore, the remarkable tyrosinase inhibitory activity of the present invention could be confirmed.

### <Experimental Example 3> Evaluation of whitening effects through measurement of melanin production inhibitory ability

In order to investigate the melanin production inhibitory ability, mouse melanoma B16F10 cells were dispensed in a 6-well plate to reach to 1×10⁵ cells, and cultured in an incubator at 37°C, 5% CO₂ for 24 hours. Then, after removing the culture medium, 2 mL of a medium in which the products of Examples 1 to 3 and Comparative Examples 1 to 3 were introduced at 40 ppm and 80 ppm, respectively, was added, followed by culturing for 3 hours. Thereafter, the product was treated with 2 nM α-MSH stimulant and cultured for 3 hours. This process is shown in FIG. 6.

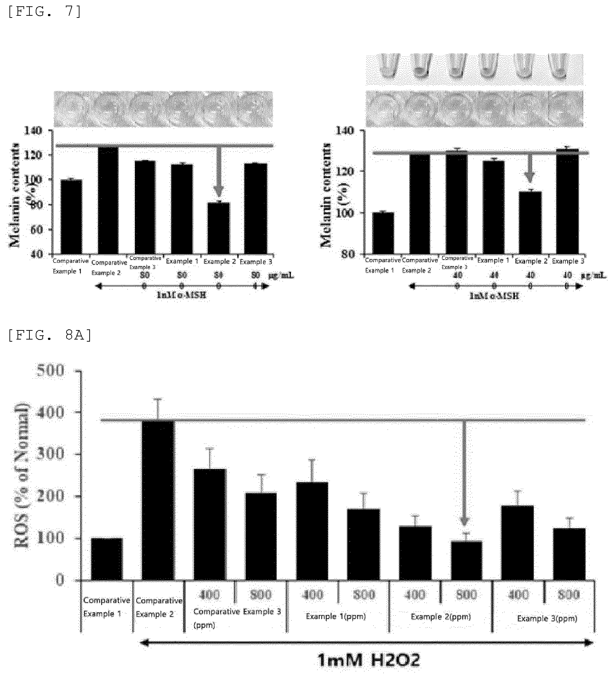

After removing the culture medium, the product was washed with PBS, scrapped and centrifuged at 4 °C and 14,000 rpm for 20 minutes. Then, 50 µL of 1N NaOH solution was added to the pellets, followed by a reaction at 60 °C for 1 hour and measurement of absorbance at 405 nm. Measurement results are shown in FIG. 7.

As a result of the experiment, in the case of Comparative Example 2, 1260 of melanin was produced compared to Comparative Example 1. Further, as compared to Comparative Example 1, 1300 of melanin was produced when treated with Comparative Example 3 at 40 ppm, while 120 % of melanin was produced when treated with the same at 80 ppm. Further, Examples 1 to 3 showed a lower melanin production rate than Comparative Examples 1 to 3. In particular, when Example 2 was used for treatment at 40 ppm, the melanin production rate was higher than that of Comparative Example 1 but significantly lower than those of other comparative examples as well as examples. Further, when treated at 80 ppm, 810 of melanin was generated compared to Comparative Example 1, therefore, it was confirmed that the melanin production was significantly inhibited at a level of 46% compared to Comparative Example 2, and at a level of about 20% compared to Comparative Example 1 of the untreated group, respectively.

### <Experimental Example 4> Evaluation of anti-aging effects

In order to evaluate the anti-aging effects of the inventive composition, active oxygen was induced with H₂O₂, and an experiment for the inhibitory effect to the generation of active oxygen was conducted. The specific experimental method is as follows.

B16F10 melanocytes and CCRF S-180II cells were dispensed in a 96-well plate to reach to 1×10⁴ cells/well, and cultured in an incubator at 37°C, 5% CO₂ for 24 hours. The culture product was treated with samples diluted by concentration and cultured for 12 hours. Then, after removing the culture medium, the product was washed with PBS, treated with 10 µM DCFDA solution, and cultured for 30 minutes.

Thereafter, the cells were treated with 1 mM H₂O₂ and cultured for 3 hours, followed by measuring a degree of intracellular ROS generation using a fluorescence analyzer (Excitation 485 nm/Emission 530 nm).

The present experiment was implemented by treatment using Comparative Examples 1 to 3 and Examples 1 to 3 at a concentration of 200, 400 ppm, or 400, 800 ppm, respectively.

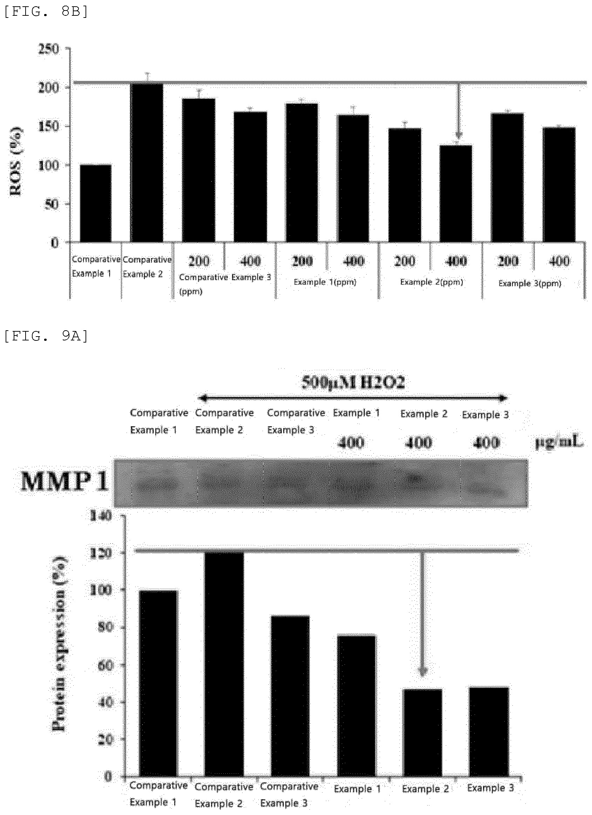

As a result of examining the active oxygen inhibitory effect of the examples on melanocytes and fibroblasts, it was found that the active oxygen increased by H₂O₂ stimulation was significantly reduced (FIGS. 8A and 8B).

### <Experimental Example 5> Evaluation of wrinkle improvement effects

In order to evaluate the wrinkle improvement effects of the inventive composition, Examples 1 to 3 and Comparative Examples 1 to 3 were used for treatment at a concentration of 400 ppm, respectively, to measure the expression levels of MMP1 and MMP8, thereby determining synthesis inhibitory effects. MMP1 and MMP8 are a type of matrix metalloproteinase (MMP) known to act on skin aging by decomposing collagen with collagenase, and specific experimental methods for the above description are as follows.

### (1) RT-PCR

CCRF S-180II cells were dispensed in a 6-well plate to reach 1×10⁵ cells/well and cultured in an incubator at 37°C, 5% CO₂. The cells were treated with samples diluted by concentration and cultured for 12 hours, followed by treatment with 1 mM H₂O₂ and culturing the same. After removing the culture medium and washing the product with PBS, total RNAs were isolated/extracted by treatment with 1 ml of trizol, dissolved in DEPC water, followed by measuring a concentration at 260 nm.

The extracted total RNAs were reacted with 10 mM dNTP mixture and oligo dT in a thermo cycler at 65 °C (PC-320, ASTEC, Japan) for 5 minutes. Then, a reaction mixture, in which 10×RT buffer, 20 mM MgCl2, 0.1MDTT and RNaseout were introduced, was added, followed by a reaction at 42 °C for 2 minutes. Subsequently, Superscript III was added, followed by further reaction at 42 °C for 50 minutes and 70°C for 15 minutes so as to synthesize cDNA. To this product, RNase H was added in the same amount as Superscript III in order to remove the remaining RNA, and the synthesized cDNA was stored at -20 °C and used for PCR experiments. The reaction mixture for PCR reaction was prepared by adding MQ-water, 10X PCR buffer containing 15 mM MgCl2, sense-primer, antisense-primer, 2.5 mM dNTP mixture and Taq polymerase. After adding the reaction mixture to cDNA and mixing the same, it was amplified using a thermocycler. PCR products were subjected to electrophoresis on agarose gel (Mupid-2plus, Advance, Japan) and then comparative analysis using GelDoc-It imaging system (UVP, USA) and image acquisition & analysis software (UVP, USA).

### (2) Western Blot

CCRF S-180II cells were dispensed in a 6-well plate to reach 1×10⁵ cells/well and cultured in an incubator at 37°C, 5% CO₂ for 24 hours. The cells were treated with samples diluted by concentration and cultured for 12 hours, followed by treatment with 1 mM H₂O₂ and culturing the sample. After removing the culture medium, the product was washed with PBS and scrapped, followed by adding RIPA lysis buffer (50 mM Tris-HCl, 150 mM NaCl, 1 mM EDTA, 1% Triton X-100, 1% sodium deoxycholate, 0.1% SDS, pH 7.4, 1 mM PMSF and protease inhibitors) thereto. Cells were lysed with lysis buffer, centrifuged at 4 °C and 14,000 rpm for 20 minutes, and then the supernatant was quantified for protein by BCA (bicinchoninic acid) protein assay (pierce BCA protein assay kit, Thermo), followed by separation of the protein through SDS-PAGE (sodium dodecyl sulfate-polyacrylamide gel) electrophoresis.

The separated protein was moved to a polyvinylidene difluoride (PVDF) membrane and blocked (5% skim milk in 1×TBST) for 1 hour. Primary antibody (1:1000) was added and left overnight at 4 °C, and then washed 3 times with 1×TBST for 15 minutes. Following this, secondary antibody (1:2000) was added to react at room temperature for 2 hours. By exposing fluorescence, which was expressed by a reaction with ECL solution in a darkroom, to an X-ray film, respective bands were identified and confirmed.

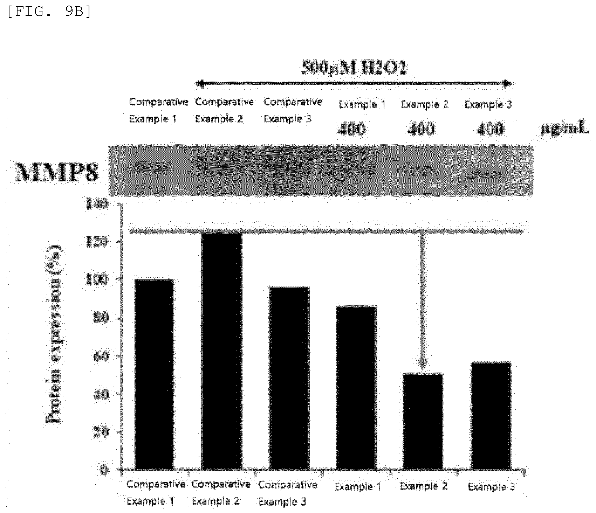

As a result of monitoring the effects of the above examples on the expression of MMP1 and MMP8 due to H₂O₂ stimulation in CCRF S-180 II cells, it was confirmed that the present examples could significantly inhibit MMP1 and MMP8 expression as compared to not only the negative control group treated with H₂O₂ but also the sample-untreated group (FIGS. 9A and 9B).

## Claims

1. A method for extracting an extract of a by-product in production of alcoholic beverages ("liquor production by-product"), comprising:
(1) drying the liquor production by-product;
(2) crushing the by-product dried in the above (1);
(3) fermenting the by-product crushed in the above (2) with any one or more strains of *Weissella cibaria, Issatchenkia orientalis* or *Saccharomyces cerevisiae;* and
(4) extracting the by-product fermented in the above (3).

2. The method according to claim 1, wherein the liquor production by-product in the above (1) is a by-product remaining after production of fermented liquor.

3. The method according to claim 1, wherein, in the drying of the above (1), a drying temperature ranges from 15 to 35 °C, and a drying time ranges from 24 to 48 hours.

4. The method according to claim 1, wherein the by-product crushed in the crushing of the above (2) has a particle size of 0.1 to 3 mm.

5. The method according to claim 1, wherein an amount of the strain used in the fermentation of the above (3) ranges from 0.005 to 3% by weight based on a total weight including the by-product and an extraction solvent.

6. The method according to claim 1, wherein, in the fermentation of the above (3), the by-product is supplemented with sugar in an amount of 0.1 to 20% by weight based on a total weight of the by-product.

7. The method according to claim 1, wherein the fermentation of the above (3) includes fermenting at 25 to 50 °C for 24 to 72 hours.

8. The method according to claim 1, wherein the extraction of the above (4) includes extracting with water or 70% (v/v) ethanol.

9. The method according to claim 1, further comprising (5) filtering the extract obtained through the extraction of the above (4), and then concentrating the same.

10. The method according to claim 9, wherein the filtered extract is concentrated at 45 to 65 °C to reach 5 to 60 brix°.

11. The method according to claim 9, further comprising (6) powdering the extract concentrated solution.

12. An extract of liquor production by-products, produced by the method according to any one of claims 1 to 11.

13. A composition for whitening, wrinkle improvement or anti-aging effects, comprising the extract according to claim 12.

14. A method for manufacturing a scrub composition, comprising:
(1) drying a liquor production by-product;
(2) crushing the by-product dried in the above (1);
(3) fermenting the by-product crushed in the above (2) with any one or more strains of *Weissella cibaria, Issatchenkia orientalis* or *Saccharomyces cerevisiae;*
(4) extracting the by-product fermented in the above (3); and
(5) pulverizing the remaining by-product after extracting in the above (4) to a particle size of 100 to 500 µm.

15. A scrub composition manufactured by the method according to claim 14.
